(19) Europäisches Patentamt
European Patent Office
Office européen des brevets

(11) EP 0 643 958 B1

(12) FASCICULE DE BREVET EUROPEEN

(45) Date de publication et mention de la délivrance du brevet:
**13.09.2000 Bulletin 2000/37**

(51) Int. Cl.[7]: **A61K 7/46**, C11B 9/00, C07C 33/12

(21) Numéro de dépôt: **94111688.1**

(22) Date de dépôt: **27.07.1994**

# (54) Utilisation en parfumerie d'isomères optiquement actifs du (E)-3,3-diméthyl-5-(2,2,3-triméthyl-3-cyclopentén-1-yl)-4-pentén-2-01

Verwendung in Parfumerie vom optisch aktiven Isomeren des (E)-3,3-Dimethyl-5-(2,2,3-trimethyl-3-cyclopenten-1-yl)-4-penten-2-ol

Use in perfumery of optically active isomers of (E)-3,3-dimethyl-5-(2,2,3-trimethyl-3-cyclopenten-1-yl-)-4-penten-2-ol

(84) Etats contractants désignés:
**CH DE ES FR GB IT LI NL**

(30) Priorité: **17.08.1993 CH 244593**

(43) Date de publication de la demande:
**22.03.1995 Bulletin 1995/12**

(73) Titulaire: **FIRMENICH SA**
**1211 Genève 8 (CH)**

(72) Inventeurs:
- **Chapuis, Christian**
  **CH-1295 Mies (CH)**
- **Gautier, Antoine**
  **Lawrenceville, New Jersey 08548 (US)**
- **Blanc, Pierre-Alain**
  **CH-1263 Crassier (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 116 903**
**EP-A- 0 155 591**
**EP-A- 0 203 528**
**EP-A- 0 466 019**
**DE-A- 1 922 391**
**GB-A- 2 024 208**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention a trait au domaine de la parfumerie. Elle concerne, plus particulièrement, l'utilisation d'isomères optiquement actifs du (E)-3,3-diméthyl-5-(2,2,3-triméthyl-3-cyclopentén-1-yl)-4-pentén-2-ol à titre d'ingrédients parfumants.

Le composé susmentionné a été décrit dans le brevet européen N° 155 591, où l'on indique qu'il possède une odeur santalée, balsamique, douce et lactée. Par ailleurs, un isomère optiquement actif de ce composé, à savoir le (-)-(E)-3,3-diméthyl-5-(2',2',3'-triméthyl-3'-cyclopentén-1'-yl)-4-pentén-2-ol, est également divulgué, mais aucune description de ses propriétés olfactives propres ne peut être trouvée dans cette référence. Au fait, il apparaît clairement que les propriétés olfactives spécifiques de chaque isomère optiquement actif étaient passées complètement inaperçues à ce jour, car il est indiqué dans ce document que les différents diastéréomères de chaque composé décrit ne se distinguaient guère les uns des autres olfactivement. Sur la base de cette divulgation, on n'aurait donc eu aucune raison de penser que l'utilisation en parfumerie de l'un ou l'autre des isomères individuels pourrait présenter un avantage quelconque. Or nous venons de découvrir avec surprise que c'est exactement l'inverse qui se passe et que certains isomères particuliers du composé susmentionné se révèlent être des ingrédients parfumants de choix, alors que d'autres présentent moins d'intérêt pour la parfumerie.

Ainsi, un objet de la présente invention est de fournir le (+)-(1'S,E)-3,3-diméthyl-5-(2',2',3'-triméthyl-3'-cyclopentén-1'-yl)-4-pentén-2-ol caractérisé par un $[\alpha]^{20}_D$ (pur) d'environ + 18,3° et le (-)-(1'R,E)-3,3-diméthyl-5-(2',2',3'-triméthyl-3'-cyclopentén-1'-yl)-4-pentén-2-ol caractérisé par un $[\alpha]^{20}_D$ (pur) d'environ - 15,6°.

Nous avons maintenant établi que, contrairement à ce qui était indiqué dans le document de l'art antérieur cité ci-dessus, ces isomères optiquement actifs de l'alcool susmentionné ont une odeur bien caractéristique et distincte l'un de l'autre. Alors qu'ils possèdent une odeur de base commune de type santalé, l'effet olfactif qu'ils impartissent aux compositions et produits dans lesquels ils peuvent être incorporés est cependant très différent. C'est ainsi que le (-)-(1'R,E)-3,3-diméthyl-5-(2',2',3'-triméthyl-3'-cyclopentén-1'-yl)-4-pentén-2-ol développe une note santalée dont le caractère ressemble fortement à l'odeur typique du lait de santal, alors que l'odeur conférée par le (+)-(1'S,E)-3,3-diméthyl-5-(2',2',3'-triméthyl-3'-cyclopentén-1'-yl)-4-pentén-2-ol est plus sèche, plus boisée-cédrée et moins lactée que celle de son isomère susmentionné. Comme il ressort des exemples présentés plus loin, ces deux composés optiquement actifs peuvent ainsi être avantageusement utilisés pour la préparation de compositions parfumantes et articles parfumés sans que leur utilisation fasse double emploi, les parfumeurs préférant l'un ou l'autre selon la nature de l'application et la connotation odorante particulière recherchée. Il a notamment été observé que l'isomère (-)-(1'R,E) était particulièrement apprécié de certains parfumeurs pour des applications de parfumerie fine, de par l'élégance et la finesse de sa note santalée, alors que l'isomère (+)-(1'S,E) était jugé un ingrédient plus avantageux dans le parfumage dit fonctionnel, c'est-à-dire de savons, détergents et autres produits de consommation divers, en raison de la puissance odorante accrue et de la meilleure performance de sa note santalée dans ces produits.

Le fait que ces deux composés trouvent un emploi heureux en parfumerie est le résultat d'une découverte que nous venons de faire. En effet, chacun de ces composés est un mélange de deux diastéréomères et nous venons de constater, avec surprise, que ces diastéréomères se comportent aussi de façon distincte l'un de l'autre, du point de vue olfactif. En raison de sa structure, qui possède deux centres chiraux, le (E)-3,3-diméthyl-5-(2,2,3-triméthyl-3-cyclopentén-1-yl)-4-pentén-2-ol peut se présenter sous forme de quatre isomères chiraux, parmi lesquels le (+)-(1'S,2S,E) s'est révélé un ingrédient parfumant de choix. Ce composé possède en effet une note odorante santalée, très puissante, où le caractère lait de santal se trouve représenté de son mieux et accompagné d'une légère note animale. Cet isomère est au fait le composant déterminant, pour ce qui est de l'odeur, dans le (+)-(1'S,E)-3,3-diméthyl-5-(2',2',3'-triméthyl-3'-cyclopentén-1'-yl)-4-pentén-2-ol, puisque l'isomère de configuration (+)-(1'S,2R,E) développe une note santalée plus faible et moins caractéristique. Curieusement, il semblerait que c'est le centre chiral présent dans la chaîne qui est déterminant pour la performance olfactive de ces composés, puisqu'il a également été constaté que l'isomère de configuration (-)-(1'R,2S,E) possédait une odeur clairement supérieure à celle de son diastéréomère (-)-(1'R,2R,E), aussi bien du point de vue de la puissance que du caractère de la note santalée, et qu'il était responsable de l'odeur élégante et appréciée du (-)-(1'R,E)-3,3-diméthyl-5-(2',2',3'-triméthyl-3'-cyclopentén-1'-yl)-4-pentén-2-ol cité plus haut.

Comme il ressort des exemples présentés plus loin, les diastéréomères de configuration (+)-(1'S,2S,E) et (-)-(1'R,2S,E) ont été systématiquement préférés par les parfumeurs lors d'essais comparatifs d'évaluation, et l'utilisation de ces composés en parfumerie est préférée selon l'invention. De même, leurs mélanges, indépendamment des proportions relatives des deux compositions, se sont révélés des ingrédients parfumants très utiles. C'était le cas par exemple du mélange contenant des quantités équivalentes des deux composants, à savoir le (+)-(2S,E)-3,3-diméthyl-5-(2',2',3'-triméthyl-3'-cyclopentén-1'-yl)-4-pentén-2-ol.

De par leurs qualités olfactives, les composés de l'invention se prêtent au parfumage d'articles aussi divers que les parfums et eaux de toilette, les savons, les gels de douche ou bain, les shampoings ou autres articles destinés au traitement et à l'hygiène des cheveux, les préparations cosmétiques, les désodorisants corporels ou d'air ambiant, les détergents ou adoucissants textiles ou, encore, les produits d'entretien. Dans ces applications, ils peuvent être

employés soit seuls, soit, comme il est plus courant en parfumerie, en mélange avec d'autres ingrédients parfumants, des solvants ou des adjuvants d'usage courant en parfumerie et que l'homme du métier est à même de choisir en fonction de l'effet désiré et de la nature du produit à parfumer.

Les concentrations dans lesquelles les composés de l'invention peuvent être utilisés pour obtenir les effets parfumants désirés varient dans une gamme de valeurs très étendue, étant bien connu que ces valeurs dépendent et de la nature de l'article à parfumer et de l'effet odorant recherché, ainsi que de la nature des autres co-ingrédients parfumants dans une composition donnée. C'est ainsi que des concentrations de l'ordre de 1 à 5%, voire 10 ou même 20% en poids dudit composé de l'invention, par rapport au poids de la composition, sont tout à fait appropriées lorsque les composés cités sont ajoutés à des compositions parfumantes variées. Des concentrations nettement inférieures à ces valeurs peuvent être employées lorsqu'ils sont utilisés pour le parfumage d'articles divers tels que ceux cités plus haut.

Les mélanges de diastéréomères selon l'invention, à savoir les (+)-(1'S,E)- et (-)-(1'R,E)-3,3-diméthyl-5-(2',2',3'-triméthyl-3'-cyclopentén-1'-yl)-4-pentén-2-ols, ont été préparés à partir des isomères optiquement actifs appropriés de l'aldéhyde campholénique, selon la méthode décrite dans le brevet EP 155 591. On a veillé à utiliser des produits de départ de grande pureté optique, afin d'obtenir des produits finaux avec une pureté optique supérieure à 97%. Les caractères analytiques de ces produits étaient identiques à ceux décrits dans le brevet européen cité, seuls les angles de rotation optique étant différents. Ces derniers sont indiqués ci-après :

**a.** (+)-(1'S,E)-3,3-diméthyl-5-(2',2',3'-triméthyl-3'-cyclopentén-1'-yl)-4-pentén-2-ol
$[\alpha]_D^{20}$ (pur) = + 18,3°
(-)-aldéhyde campholénique (produit de départ)
$[\alpha]_D^{20}$ (pur) = -9,6°
(+)-(1'S,E)-3-méthyl-5-(2',2',3'-triméthyl-3'-cyclopentén-1'-yl)-4-pentén-2-one (produit intermédiaire)
$[\alpha]_D^{20}$ (pur) = + 1,5°
(+)-(1'S,E)-3,3-diméthyl-5-(2',2',3'-triméthyl-3'-cyclopentén-1'-yl)-4-pentén-2-one (produit intermédiaire)
$[\alpha]_D^{20}$ (pur) = + 26,4°

**b.** (-)-(1'R,E)-3,3-diméthyl-5-(2',2',3'-triméthyl-3'-cyclopentén-1'-yl)-4-pentén-2-ol
$[\alpha]_D^{20}$ (pur) = - 15,6°
(-)-(1'R,E)-3,3-diméthyl-5-(2',2',3'-triméthyl-3'-cyclopentén-1'-yl)-4-pentén-2-one (produit intermédiaire)
$[\alpha]_D^{20}$ = - 26,6° ; c=1,75, $CHCl_3$

D'autre part, l'obtention des quatre diastéréomères purs du 3,3-diméthyl-5-(2,2,3-triméthyl-3-cyclopentén-1-yl)-4-pentén-2-ol, qui sont des composés nouveaux, et leur utilisation en parfumerie, notamment celle des composés préférés selon l'invention, sont le résultat d'études laborieuses, faisant usage de procédés originaux. En effet, la préparation des deux diastéréomères présents dans chacun des composés cités ci-dessus s'est révélée particulièrement difficile. Il a notamment été impossible de les obtenir à partir des composés susmentionnés, par des techniques de séparation conventionnelles, telles que chromatographie préparative en phase liquide ou gazeuse, distillation ou encore cristallisation dans des solvants choisis.

Par ailleurs, en raison de l'encombrement stérique du groupe -OH (dû à la présence du gémdiméthyle) et de la petite taille de ce groupe, beaucoup de procédés chimiques connus et couramment utilisés dans la séparation de diastéréomères, se sont révélés ici totalement inefficaces, du point de vue de la pureté et/ou du rendement en produit désiré.

C'est donc avec surprise que nous avons découvert que ces diastéréomères pouvaient être obtenus à l'état pur et avec des rendements utiles grâce à un procédé original, caractérisé en ce qu'on traite avec un excès de Na métallique, dans l'éther, un céto-éther de formule

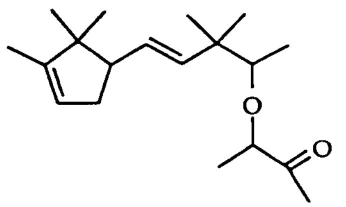

(I)

sous forme d'un isomère optiquement actif approprié, pour obtenir l'isomère correspondant du 3,3-diméthyl-5-(2,2,3-triméthyl-3-cyclopentén-1-yl)-4-pentén-2-ol.

La réaction qui caractérise le procédé de l'invention s'effectue dans les conditions décrites en détail plus loin. Les composés optiquement actifs (I), utilisés comme produits de départ dans ce procédé, sont des composés nouveaux, qui peuvent être préparés à partir de (+)-(1'S,E)- et (-)-(1'R,E)-3,3-diméthyl-5-(2',2',3'-triméthyl-3'-cyclopentén-1'-yl)-4-pentén-2-ones, à l'aide des réactions illustrées dans le schéma ci-après pour l'un de ces produits de départ :

SCHEMA I

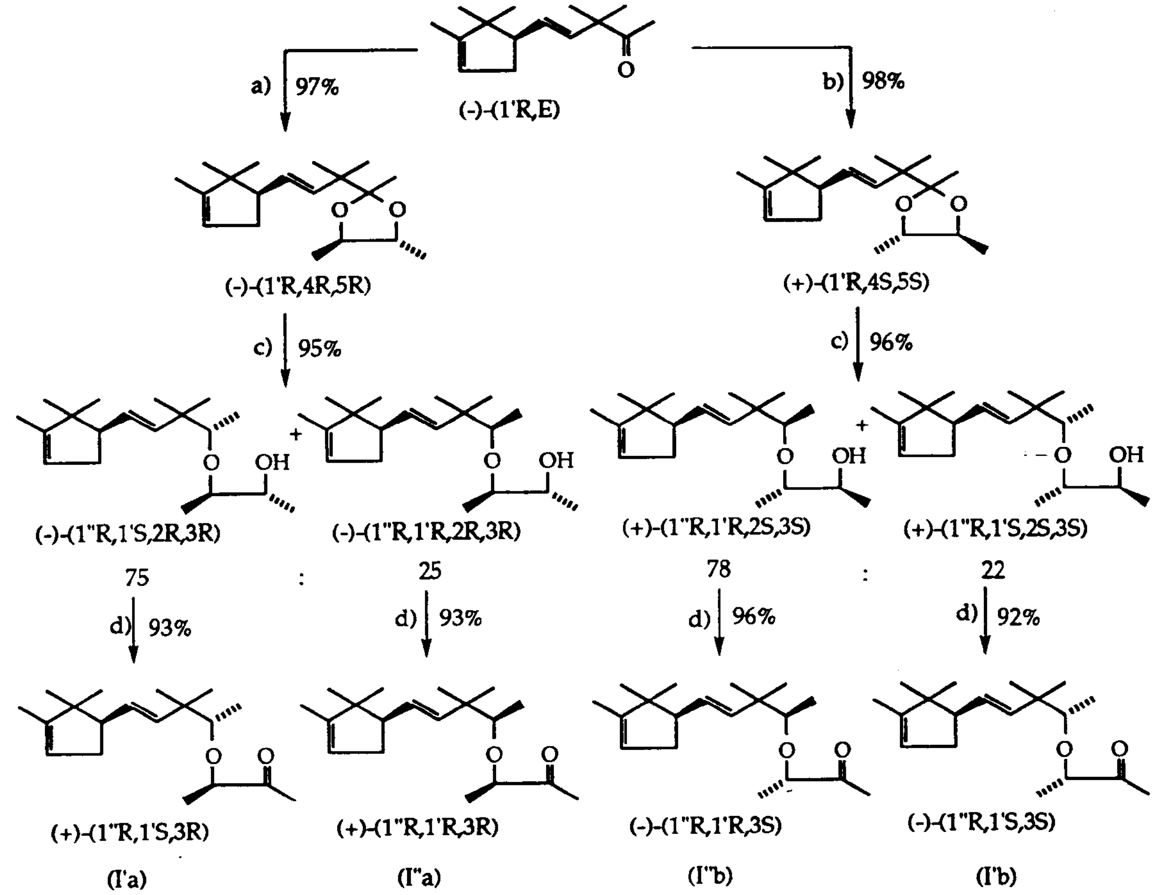


a) (-)-butanediol, acide p-toluènesulfonique, cyclohexane
b) (+)-butanediol, acide p-toluènesulfonique, cyclohexane
c) $LiAlH_4$, $AlCl_3$, éther éthylique
d) PCC (chlorochromate de pyridinium), $CH_2Cl_2$

Les cétones de départ sont transformées en les acétals chiraux représentés à l'aide de (+) ou (-)-butanediol. La réaction suivante consiste en une addition stéréosélective d'un hydrure sur les acétals susmentionnés, pour donner les hydroxyéthers représentés, sous forme de mélanges de deux diastéréomères, présents dans les proportions indiquées. Ces diastéréomères sont séparés par chromatographie et ensuite oxydés pour fournir les céto-éthers correspondants. Ces derniers ont ensuite été transformés selon l'invention, les composés de formule (I'a) et (I'b) étant des précurseurs du (-)-(1'R,2S,E)-3,3-diméthyl-5-(2',2',3'-triméthyl-3'-cyclopentén-1'-yl)-4-pentén-2-ol, et les composés de formule (I"a) et (I"b) étant des précurseurs de son isomère (-)-(1'R,2R,E).

Bien entendu, l'application de la même séquence de réactions à la (+)-(1'S,E)-3,3-diméthyl-5-(2',2',3'-triméthyl-3'-cyclopentén-1'-yl)-4-pentén-2-one permet d'obtenir les céto-éthers précurseurs des (+)-(1'S,2S,E)- et (+)-(1'S,2R,E)-3,3-diméthyl-5-(2',2',3'-triméthyl-3'-cyclopentén-1'-yl)-4-pentén-2-ols selon l'invention.

Selon le procédé de l'invention, ces quatre penténols chiraux sont obtenus avec des puretés diastéréomériques d'au moins 90%. Si désiré, ils peuvent être purifiés davantage à l'aide de procédés d'usage courant, par exemple via la formation de dérivés dont les diastéréomères ont des solubilités distinctes dans des solvants déterminés et peuvent donc être séparés l'un de l'autre par cristallisation. Un tel exemple est décrit en détail plus loin et a recours à la

préparation de dérivés dinitrobenzoates. Alternativement, on a également utilisé une méthode faisant usage de dérivés camphanoates, dont les diastéréomères pouvaient être séparés par chromatographie. L'utilisation de l'une ou l'autre de ces méthodes a été fonction de la nature du produit à purifier et notamment de son contenu en l'isomère désiré, la première desdites méthodes s'étant révélée efficace uniquement lorsque le produit à purifier était déjà enrichi en ledit isomère.

La configuration absolue de tous les produits chiraux obtenus a été déterminée à l'aide du test de Horeau [voir A. Horeau, Tetrahedron Lett. 15, 506 (1961)] et confirmée par corrélation chimique [J.S. Brooks et al., J. Chem. Soc. Perkin 1, 1974, 2114; J. Chem. Soc. Chem. Comm 1971, 1359].

L'invention concerne également un procédé alternatif pour la préparation des quatre isomères chiraux du 3,3-diméthyl-5-(2,2,3-triméthyl-3-cyclopentén-1-yl)-4-pentén-2-ol, selon lequel ces diastéréomères sont obtenus à partir des mélanges de diastéréomères décrits plus haut, à savoir les (-)-(1'R,E)-et (+)-(1'S,E)-3,3-diméthyl-5-(2',2',3'-triméthyl-3'-cyclopentén-1'-yl)-4-pentén-2-ol, par estérification sous forme d'acétate ou de chloroacétate, hydrolyse enzymatique subséquente du produit obtenu à l'aide d'une lipase appropriée et séparation des produits désirés.

La réaction d'hydrolyse s'effectue à une température comprise entre 25° et 40°C et à un pH dans une gamme de valeurs comprises entre 5 et 8. En tant que lipase, on a utilisé une lipase disponible dans le commerce. Des exemples de telles lipases sont les enzymes désignées couramment comme triacylglycérol acylhydrolases EC 3.1.1.3, par exemple les lipases pancréatiques de porc (PPL) vendues par Sigma Chem. Co. sous les noms commerciaux de Lipase Type VI ou Lipase n° L-3126, Type II. Ces lipases sont capables d'hydrolyser sélectivement un seul des diastéréomères du chloroacétate formé précédemment, laissant l'autre non réagi. Les deux produits de l'hydrolyse sont ensuite séparés par chromatographie.

Ce procédé de l'invention est défini aux revendications et décrit en détail plus loin.

L'invention sera maintenant décrite plus en détail à l'aide des exemples suivants dans lesquels les températures sont indiquées en degrés centigrades et les abréviations ont le sens usuel dans l'art.

Exemple 1

Préparation du (-)-(1'R,2R,E)-3,3-diméthyl-5-(2',2',3'-triméthyl-3'-cyclopentén-1'-yl)-4-pentén-2-ol

**a.** A partir du (-)-(1'R,E)-3,3-diméthyl-5-(2',2',3'-triméthyl-3'-cyclopentén-1'-yl)-4-pentén-2-ol (2R/2S 1 : 1 ; $[\alpha]^{20}_D$ (pur) = - 15,6°).

Ce composé (26,8 mmole) a été estérifié à l'aide de $ClCH_2COCl$ (2,1 ml; 31,9 mmole), dans la pyridine (2,1 ml), pour fournir le chloroacétate de (-)-(1'R,E)-1,2,2-triméthyl-(2',2',3'-triméthyl-3'-cyclopentén-1'-yl)-3-butényle ($[\alpha]^{20}_D$ (pur) = - 15,8°). Ce dernier a ensuite été hydrolysé à 36° à l'aide de 8 g de PPL(Lipase L-3126, Type II ; origine : Sigma Chem. Co.), dans 400 ml d'une solution 0,1 M de phosphate à pH 7,5. Après 24 h, le mélange a été extrait à l'éther, séché sur $MgSO_4$ et évaporé. Ces opérations ont été répétées trois fois pour fournir 20 g d'une huile qui a été purifiée par chromatographie sur $SiO_2$ avec cyclohexane/acétate d'éthyle 83 : 17 pour donner 13,8 g de chloroacétate de (-)-(1'R,1S,E)-1,2,2-triméthyl-4-(2',2',3'-triméthyl-3'-cyclopentén-1'-yl)-3-butényle (non réagi) et 4,75 g de (-)-(1'R,2R,E)-3,3-diméthyl-5-(2',2',3'-triméthyl-3'-cyclopentén-1'-yl)-4-pentén-2-ol désiré ($[\alpha]^{20}_D$ = - 25,4° ; c=3,03, $CHCl_3$ ; excès diastéréomérique (d.e.) 90%).)

Le chloroacétate susmentionné présentait les caractères analytiques suivants :

$[\alpha]^{20}_D$ = - 16,13° ; c=4,84, $CHCl_3$

| | |
|---|---|
| IR : | 2900, 1760, 1460, 1380, 1360, 1290, 1190, 1110, 1050, 980 $cm^{-1}$ |
| RMN($^1$H,360MHz) : | 0,73(s, 3H) ; 0,95(s, 3H) ; 1,03(s, 6H) ; 1,18(d, J=7, 3H) ; 1,61(q, J=2, 3H) ; 2,01-2,12(m, 1H) ; 2,18-2,28(m, 1H) ; 2,33(q, J=7, 1H) ; 4,04(s, 1H) ; 4,84(q, J=7, 1H) ; 5,23(large s, 1H) ; 5,41(d, J=16, 1H) ; 5,48(dd, J=7, 16, 1H) δ ppm |
| RMN($^{13}$C) : | 12,7(q) ; 15,2(q) ; 20,5(q) ; 23,4(q) ; 23,9(q) ; 25,4(q) ; 35,5(t) ; 39,8(s) ; 41,2(t) ; 48,1(s) ; 54,3(d) ; 79,3(d) ; 121,5(d) ; 129,9(d) ; 136,1(d) ; 148,05(s) ; 166,9(s) δ ppm |
| SM : | 298($M^+$,14), 204(13), 189(12), 177(37), 135(20), 121(56), 109(74), 91(30), 77(69), 69(100), 55(22), 41(28) |

Le penténol susmentionné (4,44 g ; 20 mmole) a été purifié davantage par estérification à l'aide de chlorure de 3,5-dinitrobenzoyle (6,92 g ; 24 mmole) dans la pyridine (20 ml) et l'heptane (80 ml) pendant 5 h, à 25°. Après extraction à l'éther et lavage à neutralité, on a séché et évaporé. Le produit ainsi obtenu a été chromatographié sur $SiO_2$ (éluant : 97 : 3 cyclohexane/acétate d'éthyle) et recristallisé dans l'éthanol à reflux pour fournir le 3,5-dinitrobenzoate de (-)-(1'R,1R,E)-1,2,2-triméthyl-4-(2',2',3'-triméthyl-3'-cyclopentén-1'-yl)-3-butényle dont les caractères analytiques étaient les suivants :
P. f. = 79-80°
$[\alpha]^{20}_D$ = - 40,4° ; c=0,83, $CHCl_3$

| | |
|---|---|
| IR : | 3100, 3040, 2980, 1730, 1625, 1545, 1350, 1280, 1180, 1080 $cm^{-1}$ |
| RMN($^1$H,360MHz) : | 0,73(s, 3H) ; 0,94(s, 3H) ; 1,05(s, 3H) ; 1,06(s, 3H) ; 1,34(d, J=7, 3H) ; 1,60(q, J=2, 3H) ; 2,01(m, 1H) ; 2,17(m, 1H) ; 2,38(q, J=7, 1H) ; 5,11(q, J=7, 1H) ; 5,21(large s, 1H) ; 5,52(m, 2H) ; 9,12(d, J=2, 2H) ; 9,22(t, J=2, 1H) δ ppm |
| RMN($^{13}$C) : | 12,7(q) ; 15,3(q) ; 18,45(q) ; 20,5(q) ; 23,9(q) ; 25,4(q) ; 35,5(t) ; 40,1(s) ; 48,15(s) ; 54,3(d) ; 80,2(d) ; 121,4(d) ; 122,2(d) ; 129,3(2d) ; 130,45(d) ; 134,6(s) ; 136,0(d) ; 148,1(s) ; 148,7(s) ; 162,0(s) δ ppm |
| SM : | 416($M^+$,40), 401(10), 195(40), 177(100), 149(23), 109(22), 69(21) |

Les cristaux ainsi obtenus (4,4 g ; 10,58 mmole) ont été saponifiés à l'aide de 710 mg (12,7 mmole) de KOH et 3 ml d'éthanol, le mélange étant porté à reflux pendant 30 min. On a extrait le mélange refroidi à l'éther, lavé à neutralité avec $H_2O$, séché et évaporé. Après distillation au four à boules (100°/4 Pa), on a obtenu le (-)-(1'R,2R,E)-3,3-diméthyl-5-(2',2',3'-triméthyl-3'-cyclopentén-1'-yl)-4-pentén-2-ol avec les caractères analytiques suivants :
$[\alpha]^{20}_D$ = - 26,6° ; c=4,72, $CHCl_3$ ; d.e. 94%

| | |
|---|---|
| RMN($^1$H,360MHz) : | 0,73(s, 3H) ; 0,95(s, 3H) ; 1,0(s, 3H) ; 1,01(s, 3H) ; 1,12(d, J=7, 3H) ; 1,55(large s, 1H) ; 1,61(q, J=2, 3H) ; 2,05-2,13(m, 1H) ; 2,2-2,3(m, 1H) ; 2,38(q, J=7, 1H) ; 3,49(q, J=7, 1H) ; 5,23(large s, 1H) ; 5,39(d, J=16, 1H) ; 5,5(dd, J=7, 16, 1H) δ ppm |
| RMN($^{13}$C) : | 12,7(q) ; 17,4(q) ; 20,5(q) ; 22,1(q) ; 24,0(q) ; 25,4(q) ; 35,55(t) ; 41,0(s) ; 48,1(s) ; 54,3(d) ; 74,2(d) ; 121,5(d) ; 130,5(d) ; 137,5(d) ; 148,0(s) δ ppm |

**b.** A partir de (-)-(1"R,1'R,3S,E)- ou (+)-(1"R,1'R,3R,E)-3-[1',2',2'-triméthyl-4'-(2",2",3"-triméthyl-3"-cyclopentén-1"-yl)-3'-buténytoxy]-2-butanone
Une suspension de la butanone de configuration (-)-(1"R,1'R,3S,E) (800 mg, 2,74 mmole) et de sodium (800 mg ; 33,3 atg) dans l'éther anhydre (10 ml) a été agitée pendant 4 jours. Après filtration sur $SiO_2$, on a évaporé et chromatographié pour obtenir le (-)-(1'R,2R,E)-3,3-diméthyl-5-(2',2',3'-triméthyl-3'-cyclopentén-1'-yl)-4-pentén-2-ol (rend. 49%) avec les caractères indiqués ci-dessus, à l'exception de :
$[\alpha]^{20}_D$ = - 22,1° ; c=1,05, $CCl_4$ ; d.e. 80%
Un produit similaire est obtenu lorsqu'on utilise la butanone de configuration (+)-(1"R,1'R,3R,E) comme produit de départ. Les alcools ainsi obtenus peuvent être purifiés davantage comme il est décrit sous a.

Les butanones de départ ont été préparées comme suit.

**A.** (+)-(1'R,4S,5S,E)- et (-)-(1'R,4R,5R,E)-2-[1,1-diméthyl-3-(2',2',3'-triméthyl-3'-cyclopentén-1'-yl)-2-propényl]-2,4,5-triméthyl-1,3-dioxolane
485 mg (2,2 mole) de (-)-(1'R,E)-3,3-diméthyl-5-(2',2',3'-triméthyl-3'-cyclopentén-1'-yl)-4-pentén-2-one ($[\alpha]^{20}_D$ = -26,6° ; c=1,75, $CHCl_3$) et 180 mg de (+)-(2S,3S)-butanediol (2,0 mmole) ont été portés à reflux dans 15 ml de cyclohexane avec 10 mg d'acide p-toluènesulfonique et un séparateur d'eau. Après 5 h, on a évaporé le mélange réactionnel sous vide et chromatographié le résidu sur $SiO_2$ (éluant : cyclohexane/acétate d'éthyle 97 : 3) pour obtenir le dioxolane de configuration (+)-(1'R,4S,5S,E), lequel présentait les caractères suivants (rend. 98%) :
$[\alpha]^{20}_D$ = + 9,0° ; c=1,0, $CHCl_3$

| | |
|---|---|
| IR : | 3040, 3000, 2900, 1470, 1450, 1390, 1250, 1190, 1130, 1110, 1000, 800 $cm^{-1}$ |
| RMN($^1$H,360MHz) : | 0,75(s, 3H) ; 0,94(s, 3H) ; 1,07(s, 6H) ; 1,21(d, J=7, 3H) ; 1,26(d, J=7, 3H) ; 1,28(s, 3H) ; 1,61(d, J=2, 3H) ; 2,0-2,12(m, 1H) ; 2,2-2,3(m, 1H) ; 2,38(q, J=8, 1H) ; 3,48-3,55(m, 1H) ; 3,62-3,70(m, 1H) ; 5,23(large s, 1H) ; 5,45(dd, J=8, 12, 1H) ; 5,62(d, J=12, 1H) δ ppm |
| RMN($^{13}$C) : | 12,7(q) ; 15,95(q) ; 17,7(q) ; 20,5(q) ; 22,0(q) ; 22,75(q) ; 22,9(q) ; 25,5(q) ; 35,6(t) ; 44,65(s) ; 48,25(s) ; 54,3(d) ; 77,8(d) ; 79,5(d) ; 112,4(s) ; 121,6(d) ; 128,8(d) ; 137,4(d) ; 148,15(s) δ ppm |
| SM : | 292($M^+$,0), 277(1), 115(100), 73(10), 55(10), 43(42). |

En procédant de façon identique, mais en utilisant du (-)-(2R,3R)-butanediol (180 mg), on a obtenu le dioxolane de configuration (-)-(1'R,4R,5R,E) susmentionné qui présentait les caractères suivants (rend. 97%) :
$[\alpha]^{20}_D$ = - 36,1° ; c=1,2, $CHCl_3$

IR : 3040, 3000, 2900, 1470, 1450, 1390, 1250, 1190, 1130, 1110, 1000, 800 $cm^{-1}$
RMN($^1$H,360MHz) : 0,73(s, 3H) ; 0,95(s, 3H) ; 1,05(s, 3H) ; 1,06(s, 3H) ; 1,21(d, J=7, 3H) ; 1,26(d, J=7, 3H) ; 1,28(s, 6H) ; 1,62(q, J=2, 3H) ; 2,01-2,11(m, 1H) ; 2,20-2,30(m, 1H) ; 2,39(q, J=8, 1H) ; 3,45-3,55(m, 1H) ; 3,65-3,72(m, 1H) ; 5,22(large s, 1H) ; 5,44(dd, J=8, 12, 1H) ; 5,65(d, J=12 ; 1H) δ ppm
RMN($^{13}$C) : 12,7(q) ; 16,0(q) ; 17,7(q) ; 20,5(q) ; 22,0(q) ; 22,65(q) ; 23,0(q) ; 25,5(q) ; 35,55(t) ; 44,65(s) ; 48,2(s) ; 54,2(d) ; 77,8(d) ; 79,5(d) ; 112,4(s) ; 121,6(d) ; 128,6(d) ; 137,5(d) ; 148,1(s) δ ppm
SM : 292($M^+$,0), 277(1), 115(100), 73(10), 55(10), 43(41).

**B.** (-)-(1"R,1'S,2R,3R,E)- et (-)-(1"R,1'R,2R,3R,E)-3-[1',2',2'-triméthyl-4'-(2",2",3"-triméthyl-3"-cyclopentén-1"-yl)-3'-butényloxy]-2-butanol

390 mg (10,27 mmole) de $LiAlH_4$ dans 20 ml d'éther anhydre ont été refroidis à -25°. On a ajouté goutte à goutte une solution de (-)-(1'R,4R,5R,E)-2-[1,1-diméthyl-3-(2',2',3'-triméthyl-3'-cyclopentén-1'-yl)-2-propényl]-2,4,5-triméthyl-1,3-dioxolane (600 mg ; 2,05 mmole) et $AlCl_3$ (1370 mg ; 10,27 mmole) dans 20 ml d'éther, sous azote et agitation magnétique. Après 26 h, on a lavé à neutralité avec $NaHCO_3$ pour obtenir un mélange des butanols désirés [(-)-(1"R,1'S,2R,3R)/(-)-(1"R,1'R,2R,3R) 3 : 1]. Ces composés ont été séparés par chromatographie ($SiO_2$, cyclohexane/acétate d'éthyle 97 : 3 à 9 : 1) pour fournir les composés désirés avec les caractères suivants :
<u>(-)-(1"R,1'S,2R,3R,E)</u> (pureté 92% ; rend. 48%)
$[\alpha]^{20}_D$ = - 38,8° ; c=1,0, $CCl_4$

IR : 3500, 2960, 1460, 1380, 1260, 1100, 980, 790 $cm^{-1}$
RMN($^1$H,360MHz) : 0,73(s, 3H) ; 0,95(s, 3H) ; 1,01(s, 6H) ; 1,08(d, J=7, 3H) ; 1,09(d, J=7, 3H) ; 1,23(d, J=7, 3H) ; 1,61(q, J=2, 3H) ; 2,07(m, 1H) ; 2,23(m, 1H) ; 2,38(q, J=8, 1H) ; 2,83(d, J=2, 1H, OH) ; 3,21(m, 1H) ; 3,25(m, 1H) ; 3,52(m, 1H) ; 5,22(large s, 1H) ; 5,45(m, 1H) ; 5,52(d, J=16, 1H) δ ppm
RMN($^{13}$C) : 12,7(q) ; 16,9(q) ; 17,6(q) ; 18,7(q) ; 20,5(q) ; 23,9(q) ; 24,25(q) ; 25,4(q) ; 35,4(t) ; 41,2(s) ; 48,0(s) ; 54,2(d) ; 72,0(d) ; 80,6(d) ; 82,5(d) ; 121,55(d) ; 128,7(d) ; 138,2(d) ; 148,1(s) δ ppm
SM : 294($M^+$,0), 205(2), 117(26), 73(100), 55(20)

<u>(-)-(1"R,1'R,2R,3R,E)</u> (pureté 95%; rend. 17%)
$[\alpha]^{20}_D$ = - 52,4° ; c=0,25, $CCl_4$

IR : 3500, 2960, 1460, 1380, 1260, 1100, 980, 790 $cm^{-1}$
RMN($^1$H,360MHz) : 0,73(s, 3H) ; 0,93(s, 3H) ; 1,02(s, 3H) ; 1,03(s, 3H) ; 1,06(d, J=7, 3H) ; 1,08(d, J=7, 3H) ; 1,14(d, J=7, 3H) ; 1,61(q, J=2, 3H) ; 2,06(m, 1H) ; 2,22(m, 1H) ; 2,34(m, 1H) ; 2,78(large s, 1H, OH) ; 3,20(m, 2H) ; 3,50(m, 1H) ; 5,22(large s, 1H) ; 5,42(m, 2H) δ ppm
RMN($^{13}$C) : 12,75(q) ; 14,4(q) ; 15,5(q) ; 18,6(q) ; 20,5(q) ; 23,0(q) ; 24,95(q) ; 25,4(q) ; 35,65(t) ; 40,3(s) ; 48,15(s) ; 54,4(d) ; 71,2(d) ; 76,7(d) ; 78,6(d) ; 121,6(d) ; 128,5(d) ; 137,9(d) ; 148,1(s) δ ppm
SM : 294($M^+$,0), 205(2), 117(24), 73(100), 55(20)

**C.** (+)-(1"R,1'R,2S,3S,E)- et (+)-(1"R,1'S,2S,3S,E)-3-[1',2',2'-triméthyl-4'-(2",2",3"-triméthyl-3"-cyclopentén-1"-yl)-3'-buténylox]-2-butanol

Préparés de façon analogue à celle décrite sous B. mais en utilisant le (+)-(1'R,4S,5S,E)-2-[1,1-diméthyl-3-(2',2',3'-triméthyl-3'-cyclopentén-1'-yl)-2-propényl]-2,4,5-triméthyl-1,3-dioxolane comme produit de départ.
<u>(+)-(1"R,1'R,2S,3S,E)</u> (pureté 90%; rend. 52%)
$[\alpha]^{20}_D$ = + 10,4° ; c=1,2, $CCl_4$

IR : 3500, 2960, 1460, 1380, 1260, 1100, 980, 790 $cm^{-1}$
RMN($^1$H,360MHz) : 0,73(s, 3H) ; 0,92(s, 3H) ; 0,99(s, 3H) ; 1,02(s, 3H) ; 1,09(d, J=7, 6H) ; 1,11(d, J=7, 3H) ; 1,60(q, J=2, 3H) ; 2,07(m, 1H) ; 2,2-2,4(m, 2H) ; 2,88(large s, 1H, OH) ; 3,19(m, 1H) ; 3,24(m, 1H) ; 3,52(m, 1H) ; 5,23(large s, 1H) ; 5,45(m, 1H) δ ppm
RMN($^{13}$C) : 12,7(q) ; 16,75(q) ; 17,7(q) ; 18,7(q) ; 20,5(q) ; 23,2(q) ; 24,2(q) ; 25,4(q) ; 35,7(t) ; 41,3(s) ; 48,2(s) ; 54,4(d) ; 72,0(d) ; 80,8(d) ; 82,3(d) ; 121,6(d) ; 128,9(d) ; 138,7(d) ; 148,0(s) δ ppm
SM : 294($M^+$,0), 117(34), 73(100), 55(16)

(+)-(1"R,1'S,2S,3S) (pureté 98%; rend. 15%)
$[\alpha]^{20}_D$ = + 34,7°; c=1,3, $CCl_4$

IR : 3500, 2960, 1460, 1380, 1260, 1100, 980, 790 $cm^{-1}$
RMN($^1$H,360MHz) : 0,74(s, 3H) ; 0,94(s, 3H) ; 0,99(s, 3H) ; 1,00(s, 3H) ; 1,02(d, J=7, 3H) ; 1,04(d, J=7, 3H) ; 1,14(d, J=7, 3H) ; 1,61(q, J=2, 3H) ; 2,08(m, 1H) ; 2,22(m, 1H) ; 2,35(m, 1H) ; 2,79(large s, 1H, OH) ; 3,19(m, 2H) ; 3,5(m, 1H) ; 5,22(large s, 1H) ; 5,43(m, 2H) δ ppm
RMN($^{13}$C) : 12,75(q) ; 14,4(q) ; 15,45(q) ; 18,6(q) ; 20,5(q) ; 23,4(q) ; 24,8(q) ; 25,4(q) ; 35,6(t) ; 40,25(s) ; 48,1(s) ; 54,4(d) ; 71,2(d) ; 76,5(d) ; 78,7(d) ; 121,55(d) ; 128,45(d) ; 137,9(d) ; 148,15(s) δ ppm
SM : 294($M^+$,0), 117(34), 73(100), 55(14)

**D.** (-)-(1"R,1'R,3S,E)-, (-)-(1"R,1'S,3S,E)- ; (+)-(1"R,1'R,3R,E)- et (+)-(1"R,1'S, 3R,E)-3-[1',2',2'-triméthyl-4'-(2",2",3"-triméthyl-3"-cyclopentén-1"-yl)-3'-buténylоxy]-2-butanone

Ces butanones ont été obtenues par oxydation des hydroxy-butanols appropriés (voir Schéma I) décrits sous B. et C., à l'aide de PCC, dans le dichlorométhane. Tous les produits obtenus ont été purifiés par chromatographie sur $SiO_2$ (cyclohexane/acétate d'éthyle 97 : 3) et obtenus avec un rendement supérieur à 92%.

(+)-(1"R,1'S,3R,E) (pureté 92%)
$[\alpha]^{20}_D$ = + 13,3° ; c=1,0, $CCl_4$

IR : 2960, 1720, 1460, 1370, 1360, 1110, 980 $cm^{-1}$
RMN($^1$H,360MHz) : 0,72(s, 3H) ; 0,93(s, 3H) ; 1,03(s, 6H) ; 1,06(d, J=7, 3H) ; 1,27(d, J=7, 3H) ; 1,61(q, J=2, 3H) ; 2,05(m, 1H) ; 2,19(s, 3H) ; 2,20(m, 1H) ; 2,35(m, 1H) ; 3,17(q,J=7, 1H) ; 3,85(q, J=7, 1H) ; 5,23(large s, 1H) ; 5,45(m, 2H) δ ppm
RMN($^{13}$C) : 12,7(q) ; 15,4(q) ; 18,4(q) ; 20,5(q) ; 23,2(q) ; 24,8(q) ; 25,2(q) ; 25,4(q) ; 35,5(t) ; 40,9(s) ; 48,1(s) ; 54,4(d) ; 80,25(d) ; 82,2(d) ; 121,5(d) ; 128,5(d) ; 137,95(d) ; 148,1(s) ; 211,9(s) δ ppm
SM : 292($M^+$,0), 115(100), 71(35), 43(16)

(+)-(1"R,1'R,3R,E) (pureté 95%)
$[\alpha]^{20}_D$ = + 35,1° ; c=0,2, $CCl_4$

IR : 2960, 1720, 1460, 1370, 1360, 1110, 980 $cm^{-1}$
SM : 292($M^+$,0), 115(100), 71(26), 43(16)

(-)-(1"R,1'S,3S,E) (pureté 90%)
$[\alpha]^{20}_D$ = - 4,8° ; c=1,3, $CCl_4$

IR : 2960, 1720, 1460, 1370, 1360, 1110, 980 $cm^{-1}$
RMN($^1$H,360MHz) : 0,74(s, 3H) ; 0,94(s, 3H) ; 1,02(s, 6H) ; 1,03(d, J=7, 3H) ; 1,23(d, J=7, 3H) ; 1,61(q, J=2, 3H) ; 2,08(m, 1H) ; 2,19(s, 3H) ; 2,21(m, 1H) ; 2,35(m, 1H) ; 3,21 (q, J=7, 1H) ; 3,79(q, J=7, 3H) ; 5,23(large s, 1H) ; 5,45(m, 2H) δ ppm
RMN($^{13}$C) : 12,7(q) ; 14,7(q) ; 16,8(q) ; 20,5(q) ; 23,4(q) ; 24,6(q) ; 25,4(2q) ; 29,7(t) ; 35,6(t) ; 40,5(s) ; 48,1(s) ; 54,45(d) ; 79,85(d) ; 81,9(d) ; 121,5(d) ; 128,5(d) ; 137,8(d) ; 148,15(s) ; 212,4(s) δ ppm
SM : 292($M^+$,0), 115(100), 71(30), 43(13)

(-)-(1"R,1'R,3S,E) (pureté 98%)
$[\alpha]^{20}_D$ = - 37,5° ; c=1,6, $CCl_4$

IR : 2960, 1720, 1460, 1370, 1360, 1110, 980 $cm^{-1}$
RMN($^1$H,360MHz) : 0,73(s, 3H) ; 0,93(s, 3H) ; 1,01(s, 3H) ; 1,02(s, 3H) ; 1,06(d, J=7, 3H) ; 1,26(d, J=7, 3H) ; 1,61(q, J=7, 3H) ; 2,07(m, 1H) ; 2,21(s, 3H) ; 2,23(m, 1H) ; 2,35(m, 1H) ; 3,18(q, J=7, 1H) ; 3,83(q, J=7, 1H) ; 5,22(large s, 1H) ; 5,43(m, 2H) δ ppm
RMN($^{13}$C) : 12,7(q) ; 15,45(q) ; 18,4(q) ; 20,5(q) ; 23,2(q) ; 24,5(q) ; 25,3(q) ; 25,4(q) ; 35,6(t) ; 40,95(s) ; 48,2(s) ; 54,4(d) ; 80,4(d) ; 82,1(d) ; 121,5(d) ; 128,65(d) ; 138,05(d) ; 148,1(s) ; 211,9(s) δ ppm
SM : 292($M^+$,0), 115(100), 71(42), 55(12), 43(20)

Exemple 2

Préparation du (-)-(1'R,2S,E)-3,3-diméthyl-5-(2',2',3'-triméthyl-3'-cyclopentén-1'-yl)-4-pentén-2-ol

**a.** A partir de (-)-(1'R,E)-3,3-diméthyl-5-(2',2',3'-triméthyl-3'-cyclopentén-1'-yl)-4-pentén-2-ol

Le chloroacétate non réagi obtenu selon l'Exemple 1a. (11,7 mmole) a été saponifié à l'aide de KOH (14,3 mmole) dans l'éthanol (10 ml) à reflux, pendant 30 min. Après extraction à l'éther, on a obtenu l'alcool désiré ($[\alpha]^{20}_D$ = - 7,17° ; c=3,9, $CHCl_3$).

Ce produit a été purifié selon la méthode décrite dans l'exemple 1a. en passant par le 3,5-dinitrobenzoate de (+)-(1'R,1S,E)-1,2,2-triméthyl-4-(2',2',3'-triméthyl-3'-cyclopentén-1'-yl)-3-butényle, dont la recristallisation dans l'éthanol à -20° a fourni un produit dont les caractères analytiques étaient :

$[\alpha]^{20}_D$ = + 5,75° ; c=2,44, $CHCl_3$

| | |
|---|---|
| IR : | 3100, 3040, 2980, 1730, 1625, 1545, 1350, 1280, 1180, 1080 $cm^{-1}$ |
| RMN($^1$H,360MHz) : | 0,69(s, 3H) ; 0,90(s, 3H) ; 1,13(s, 6H) ; 1,34(d, J=7, 3H) ; 1,59(q, J=2, 3H) ; 2,05-2,15(m, 1H) ; 2,22-2,30(m, 1H) ; 2,39(q, J=7, 1H) ; 5,12(q, J=7, 1H) ; 5,23(large s, 1H) ; 5,52(m, 2H) ; 9,12(d, J=2, 2H) ; 9,22(t, J=2, 1H) δ ppm |
| RMN($^{13}$C) : | 12,7(q) ; 15,35(q) ; 20,5(q) ; 23,9(2q) ; 25,35(q) ; 35,6(t) ; 40,0(s) ; 48,1(s) ; 54,4(d) ; 80,2(d) ; 121,5(d) ; 122,2(d) ; 129,3(2d) ; 130,5(d) ; 134,6(s) ; 135,9(d) ; 148,0(s) ; 148,7(s) ; 162,0(s) δ ppm |

La saponification de ce produit a fourni le (-)-(1'R,2S,E)-3,3-diméthyl-5-(2',2',3'-triméthyl-3'-cyclopentén-1'-yl)-4-pentén-2-ol avec les caractères suivants :

$[\alpha]^{20}_D$ (pur) = - 6,3° ; d.e. 94%

| | |
|---|---|
| RMN($^1$H,360MHz) : | 0,73(s, 3H) ; 0,95(s, 3H) ; 1,00(s, 3H) ; 1,01(s, 3H) ; 1,12(d, J=7, 3H) ; 1,5(d, J=5, 1H) ; 1,61(q,J=2, 3H) ; 2,05-2,13(m, 1H) ; 2,2-2,3(m, 1H) ; 2,38(q, J=7, 1H) ; 3,49(q, d, J=5, 7, 1H) ; 5,23(large s, 1H) ; 5,41(d, J=16, 1H) ; 5,5(dd, J=7, 16, 1H) δ ppm |
| RMN($^{13}$C) : | 12,7(q) ; 17,45(q) ; 20,6(q) ; 22,2(q) ; 24,1(q) ; 25,5(q) ; 35,55(t) ; 40,9(s) ; 48,1(s) ; 54,4(d) ; 74,3(d) ; 121,5(d) ; 130,5(d) ; 137,3(d) ; 148,0(s) δ ppm |

**b.** A partir de (+)-(1"R,1'S,3R)- ou (-)-(1"R,1'S,3S)-3-[1',2',2'-triméthyl-4'-(2",2",3"-triméthyl-3"-cyclopentén-1"-yl)-3'-buténуloxy]-2-butanone

On a procédé de façon analogue à celle décrite dans l'exemple 1b. La (+)-(1"R,1'S,3R)-butanone susmentionnée a fourni le produit désiré avec un $[\alpha]^{20}_D$ = - 8,0° ; c=0,5, $CCl_4$, 92% pureté diastéréomèrique, et la (-)-(1"R,1'S,3S)-butanone a donné l'alcool ayant un $[\alpha]^{20}_D$ = - 10,0° ; c=0,5, $CCl_4$.

Les deux butanones de départ ont été obtenues comme il est décrit dans l'exemple 1b. A-D.

Exemple 3

Préparation du (+)-(1'S,2R,E)-3,3-diméthyl-5-(2',2',3'-triméthyl-3'-cyclopentén-1'-yl)-4-pentén-2-ol

**a.** A partir de (+)-(1'S,E)-3,3-diméthyl-5-(2',2',3'-triméthyl-3'-cyclopentén-1'-yl)-4-pentén-2-ol (2R/2S 1 : 1 ; $[\alpha]^{20}_D$ (pur )= + 18,6°)

On a procédé comme il est décrit dans l'exemple 1a. en utilisant le chloroacétate de (+)-(1'S,E)-1,2,2-triméthyl-(2',2',3'-triméthyl-3'-cyclopentén-1'-yl)-3-buténуle ($[\alpha]^{20}_D$ (pur)= + 18,6° ; 5 g, 16,7 mmole ; obtenu par estérification du penténol susmentionné), 5 g de PPL (Lipase L-3126, Type II ; origine : Sigma Chem. Co.) et 5 g de tert-butanol. La chromatographie ($SiO_2$ ; cyclohexane/acétate d'éthyle 98 : 2) du produit obtenu a fourni le chloroacétate de (+)-(1'S,1S,E)-1,2,2-triméthyl-4-(2',2',3'-triméthyl-3'-cyclopentén-1'-yl)-3-buténуle, non réagi, dont les caractères étaient les suivants :

$[\alpha]^{20}_D$ = + 14,4° ; c=2,75, $CCl_4$

| | |
|---|---|
| IR : | 2900, 1760, 1460, 1380, 1360, 1290, 1190, 1110, 1050, 980 $cm^{-1}$ |

RMN($^1$H,360MHz) : 0,73(s, 3H) ; 0,94(s, 3H) ; 1,02(s, 3H) ; 1,03(s, 3H) ; 1,18(d, J=7, 3H) ; 1,61(q, J=2, 3H) ; 2,0-2,1(m, 1H) ; 2,2-2,3(m, 1H) ; 2,33(q, J=7, 1H) ; 4,03(s, 2H) ; 4,84(q, J=7, 1H) ; 5,23(large s, 1H) ; 5,39(d, J=16, 1H) ; 5,48(dd, J=7, 16, 1H) δ ppm

RMN($^{13}$C) : 12,7(q) ; 15,2(q) ; 20,5(q) ; 23,2(q) ; 23,9(q) ; 25,4(q) ; 35,6(t) ; 39,8(s) ; 41,2(t) ; 48,2(s) ; 54,3(d) ; 79,2(d) ; 121,5(d) ; 130,0(d) ; 136,2(d) ; 148,1(s) ; 166,9(s) δ ppm

SM : 298($M^+$,14), 204(13), 189(12), 177(37), 135(20), 121(56), 109(74), 91(30), 77(69), 69(100), 55(22), 41(28)

ainsi que le penténol désiré (après élution du chloroacétate, le gradiant du solvant a été varié à 96 : 4, 92 : 8 et finalement 9 : 1) avec $[\alpha]^{20}_D$ = + 6,15° ; c=1,22, $CCl_4$ ; d.e. 94%.
Les autres caractères analytiques du composé final étaient identiques à ceux de son énantiomère (-)-(1'R,2S,E) (exemple 2).

**b.** A partir de (-)-(1"S,1'R,3R)- ou (+)-(1"S,1'R,3S)-3-[1',2',2'-triméthyl-4'-(2",2",3"-triméthyl-3"-cyclopentén-1"-yl)-3'-buténylоxy]-2-butanone

On a procédé de façon similaire à celle décrite dans l'exemple 1b., les butanones de départ susmentionnées ayant été obtenues à partir de la (+)-(1'S,E)-3,3-diméthyl-5-(2',2',3'-triméthyl-3'-cyclopentén-1'-yl)-4-penten-2-one ($[\alpha]^{20}_D$ (pur) = + 26,4° ; voir plus haut) de façon analogue à celle décrite dans l'exemple 1b. A-D.

Exemple 4

Préparation du (+)-(1'S,2S,E)-3,3-diméthyl-5-(2',2',3'-triméthyl-3'-cyclopentén-1'-yl)-4-pentén-2-ol

**a.** A partir de (+)-(1'S,E)-3,3-diméthyl-5-(2',2',3'-triméthyl-3'-cyclopentén-1'-yl)-4-pentén-2-ol (2R/2S 1 : 1 ; $[\alpha]^{20}_D$ (pur) = + 18,6°)

Le chloroacétate de (+)-(1'S,1S,E)-1,2,2-triméthyl-4-(2',2',3'-triméthyl-3'-cyclopentén-1'-yl)-3-buténуle (2,7 g, 9,0 mmole ; voir exemple 3a.) a été saponifié à l'aide de NaOH (400 mg ; 10 mmole) dans 10 ml d'un mélange éthanol/eau 4 : 1 à reflux, pendant 30 min. On a évaporé sous vide, extrait à l'éther, lavé à neutralité avec eau, séché sur $MgSO_4$ et évaporé pour obtenir le produit désiré ($[\alpha]^{20}_D$ = + 22,0° ; c=1,02, $CCl_4$).
Ce produit a été ensuite purifié comme il est décrit dans l'exemple 1a., via le 3,5-dinitrobenzoate de (+)-(1'S,1S,E)-1,2,2-triméthyl-4-(2',2',3'-triméthyl-3'-cyclopentén-1'-yl)-3-buténуle dont les caractères analytiques étaient identiques à ceux de son énantiomère décrit dans l'exemple 1a., à l'exception de
$[\alpha]^{20}_D$ = + 40,8° ; c=1,08, $CCl_4$
Ce produit (0,7 g ; 1,68 mmole) a ensuite été saponifié à l'aide de NaOH (74 mg ; 1,85 mmole) dans 5 ml d'éthanol/eau 4 : 1. Après évaporation, extraction à l'éther, lavage à neutralité avec eau, séchage sur $MgSO_4$ et évaporation à sec, on a obtenu le (+)-(1'S,2S,E)-3,3-diméthyl-5-(2',2',3'-triméthyl-3'-cyclopentén-1'-yl)-4-pentén-2-ol avec $[\alpha]^{20}_D$ = + 26,9° ; c=1,3, $CHCl_3$ ; d.e. 94%.
Les autres caractères analytiques étaient identiques à ceux de son énantiomère (-)-(1'R,2R) décrit dans l'exemple 1.

**b.** A partir de (-)-(1"S,1'S,3R)- ou (+)-(1"S,1'S,3S)-3-[1',2',2'-triméthyl-4'-(2",2",3"-triméthyl-3"-cyclopentén-1"-yl)-3'-buténylоxy]-2-butanone

On a procédé de façon similaire à celle décrite dans l'exemple 1b., les butanones de départ susmentionnées ayant été obtenues à partir de la (+)-(1'S,E)-3,3-diméthyl-5-(2',2',3'-triméthyl-3'-cyclopentén-1'-yl)-4-penten-2-one ($[\alpha]^{20}_D$ (pur) = + 26,4° ; voir plus haut) de façon analogue à celle décrite dans l'exemple 1b. A-D.

Exemple 5

Essai comparatif sur mouillette

Les quatre diastéréomères du 3,3-diméthyl-5-(2,2,3-triméthyl-3-cyclopentén-1-yl)-4-pentén-2-ol ont été évalués à l'aveugle, sur mouillette, par un panel de neuf experts parfumeurs.
Les mouillettes ont été trempées dans une solution à 10% dans le dipropylèneglycol (DIPG) de chacun des quatre composés, de façon à imprégner une zone d'environ I cm de longueur sur la mouillette. Les parfumeurs ont ensuite senti les quatre mouillettes et jugé leur odeur du point de vue de la puissance et qualité de la note santalée.
Les parfumeurs ont unanimement préféré la mouillette comportant le (+)-(1'S,2S,E)-3,3-diméthyl-5-(2',2',3'-triméthyl-3'-cyclopentén-1'-yl)-4-pentén-2-ol dont l'odeur santalée a été jugée remarquablement puissante et élégante, avec un caractère lait de santal très apprécié. La mouillette qui avait été trempée dans le (-)-(1'R,2S,E)-3,3-diméthyl-5-(2',2',3'-

triméthyl-3'-cyclopentén-1'-yl)-4-pentén-2-ol a pour sa part été classée 5 fois sur 8 en deuxième place, son odeur santalée étant jugée plus faible que celle de son diastéréomère susmentionné, mais toujours très puissante, alors que trois parfumeurs avaient plutôt préféré la mouillette contenant le (+)-(1'S,2R,E)-3,3-diméthyl-5-(2',2',3'-triméthyl-3'-cyclopentén-1'-yl)-4-pentén-2-ol. Tous les huit parfumeurs ont jugé la mouillette portant le (-)-(1'R,2R,E)-3,3-diméthyl-5-(2',2',3'-triméthyl-3'-cyclopentén-1'-yl)-4-pentén-2-ol la moins intéressante, son odeur étant moins santalée et caractéristique. L'un des parfumeurs, généralement anosmique aux notes santalées, n'a détecté que l'odeur de la première mouillette mentionnée.

Exemple 6

Essai comparatif sur mouillette

Les (-)-(1R',E)- et (+)-(1'S,E)-3,3-diméthyl-5-(2',2',3'-triméthyl-3'-cyclopentén-1'-yl)-4-pentén-2-ols selon l'invention, en solution à 10% dans le dipropylène glycol (DIPG), ont été évalués à l'aveugle, sur mouillette, par un panel de 9 experts parfumeurs. Six parmi ces derniers ont préféré l'odeur de la mouillette qui avait été imprégnée avec la solution du (-)-(1'R,E)-3,3-diméthyl-5-(2',2',3'-triméthyl-3'-cyclopentén-1'-yl)-4-pentén-2-ol, en la trouvant fortement santalée, douce et lactée, plus élégante et fine que l'odeur de la mouillette portant la solution du (+)-(1'S,E)-3,3-diméthyl-5-(2',2',3'-triméthyl-3'-cyclopentén-1'-yl)-4-pentén-2-ol. Les parfumeurs ont cependant unanimement trouvé que cette dernière exhalait une odeur santalée nettement plus puissante, quoique moins lactée, plus sèche et boisée-cédrée. Ces effets étaient encore plus marqués lorsque l'on a comparé à l'aveugle les (+)-(1'S,2S,E)- et (-)-(1'R,2S,E)-3,3-diméthyl-5-(2',2',3'-triméthyl-3'-cyclopentén-1'-yl)-4-pentén-2-ols.

Exemple 7

Composition parfumante

On a préparé une composition de base destinée à un parfum féminin en mélangeant les ingrédients suivants :

| Ingrédients | Parties en poids |
|---|---|
| Acétate de benzyle | 60 |
| Acétate de géranyle | 10 |
| Acétate de styrallyle | 20 |
| Aldéhyde hexylcinnamique | 90 |
| Glycolate d'allyle amyle à 10%* | 120 |
| Ambrox® DL à 10%* 1) | 20 |
| Essence de bergamote | 500 |
| Essence de citron Sfuma | 40 |
| Citronellol | 60 |
| Coumarine | 135 |
| Dihydromyrcenol 2) | 15 |
| Galaxolide® 50 3) | 95 |
| Hedione® 4) | 730 |
| Indol à 10% * | 15 |
| Isoeugénol | 20 |
| Absolue de jasmin | 80 |
| α-Méthylionone | 540 |
| Muscone | 90 |
| Octynecarbonate de méthyle à 1%* | 30 |
| p-Crésol à 1%* | 80 |
| Phénéthylol | 40 |
| Essence de baies de piment | 30 |
| Salicylate de benzyle | 100 |
| Salicylate de pipol | 35 |
| Essence de santal | 920 |
| Undécalactone | 10 |
| Vanilline à 10% * | 95 |
| Essence d'Ylang | 160 |
| Total | 4140 |

* dans le DIPG
1) tétraméthyl-perhydronaphtofurane ; origine : Firmenich SA, Genève, Suisse
2) 2,6-diméthyl-7-octén-2-ol ; origine : IFF Inc., USA
3) 1,3,4,6,7,8-hexahydro-4,6,6,7,8,8-hexaméthyl-cyclopenta[γ]isochromène ; origine : IFF Inc., USA
4) dihydrojasmonate de méthyle ; origine : Firmenich SA, Genève, Suisse

A cette composition de base de type boisé, oriental, on a ajouté respectivement 100 parties en poids de (+)-(1'S,E)-3,3-diméthyl-5-(2',2',3'-triméthyl-3'-cyclopentén-1'-yl)-4-pentén-2-ol pour préparer une nouvelle composition A et 200 parties en poids de (-)-(1'R,E)-3,3-diméthyl-5-(2',2',3'-triméthyl-3'-cyclopentén-1'-yl)-4-pentén-2-ol pour obtenir

une nouvelle composition B. Ces deux compositions ont ensuite été évaluées à l'aveugle par un panel de 9 experts parfumeurs. L'avis de ces derniers était partagé, six ayant préféré la composition A pour son impact accru et trois ayant choisi la composition B, dont ils trouvaient la note plus élégante.

Ce partage d'avis s'est révélé encore plus profond lors d'une évaluation à l'aveugle de deux compositions préparées en ajoutant à la composition de base la même quantité, 100 parties en poids, de chacun des deux isomères susmentionnés. Alors que tous les parfumeurs étaient d'accord pour trouver que la puissance de l'odeur de la composition contenant l'isomère (+)-(1'S,E) était nettement supérieure à celle de la composition contenant l'isomère (-)-(1'R,E), ils jugeaient le caractère odorant des deux compositions trop distinct pour manifester une préférence. De leur avis, la première composition développait une odeur santalée moins fine, mais avec un caractère boisé-cédré accentué et un plus fort impact, ce qui la rendait très utile pour certains types d'applications fonctionnelles exigeant un meilleur rapport prix/performance. Quant à la deuxième composition, elle exhalait une fragrance douce et lactée très élégante, particulièrement avantageuse pour la préparation de parfums à caractère oriental. Cet effet olfactif était encore plus évident lorsqu'on a remplacé le mélange de diastéréomères (-)-(1'R,E) par l'un de ses composants, à savoir le (-)-(1'R,2S,E)-3,3-diméthyl-5-(2',2',3'-triméthyl-3'-cyclopentén-1'-yl)-4-pentén-2-ol.

De même, l'addition à la composition du (+)-(1'S,2S,E)-3,3-diméthyl-5-(2',2',3'-triméthyl-3'-cyclopentén-1'-yl)-4-pentén-2-ol renforçait encore la puissance de son odeur par rapport à celle de la composition contenant le mélange de diastéréomères (+)-(1'S,E).

**Revendications**

1. Un composé choisi dans le groupe constitué par

   a. (+)-(1'S,2R,E)-3,3-diméthyl-5-(2',2',3'-triméthyl-3'-cyclopentén-1'-yl)-4-pentén-2-ol
   b. (+)-(1'S,2S,E)-3,3-diméthyl-5-(2',2',3'-triméthyl-3'-cyclopentén-1'-yl)-4-pentén-2-ol
   c. (-)-(1'R,2R,E)-3,3-diméthyl-5-(2',2',3'-triméthyl-3'-cyclopentén-1'-yl)-4-pentén-2-ol
   d. (-)-(1'R,2S,E)-3,3-diméthyl-5-(2',2',3'-triméthyl-3'-cyclopentén-1'-yl)-4-pentén-2-ol.

2. Un composé choisi dans le groupe constitué par

   a. (+)-(2S,E)-3,3-diméthyl-5-(2',2',3'-triméthyl-3'-cyclopentén-1'-yl)-4-pentén-2-ol
   b. (-)-(2R,E)-3,3-diméthyl-5-(2',2',3'-triméthyl-3'-cyclopentén-1'-yl)-4-pentén-2-ol.

3. Utilisation à titre d'ingrédient parfumant d'un composé selon la revendication 1 ou 2.

4. Composition parfumante ou article parfumé contenant à titre d'ingrédient actif un composé selon la revendication 1 ou 2.

5. A titre d'article parfumé selon la revendication 4, un parfum ou une eau de toilette, un savon, un gel de douche ou bain, une préparation cosmétique, un shampoing, un désodorisant corporel ou d'air ambiant un détergent ou un adoucissant textile, ou un produit d'entretien.

6. Procédé pour conférer, modifier, améliorer ou augmenter le caractère odorant de type santalé-lait de santal d'une composition parfumante ou d'un article parfumé, caractérisé en ce qu'on ajoute à ladite composition ou audit article du (-)-(1'R,E)-3,3-diméthyl-5-(2',2',3'-triméthyl-3'-cyclopentén-1'-yl)-4-pentén-2-ol.

7. Procédé pour conférer, modifier, améliorer ou augmenter le caractère odorant de type santalé-boisé-cédré d'une composition parfumante ou d'un article parfumé, caractérisé en ce qu'on ajoute à ladite composition ou audit article du (+)-(1'S,E)-3,3-diméthyl-5-(2',2',3'-triméthyl-3'-cyclopentén-1'-yl)-4-pentén-2-ol.

8. Procédé pour la préparation d'un composé selon la revendication 1 ayant une configuration (1'S,E), caractérisé en ce qu'il comprend les étapes suivantes:

   a) l'estérification du (+)-(1'S,E)-3,3-diméthyl-5-(2',2',3'-triméthyl-3'-cyclopentén-1'-yl)-4-pentén-2-ol sous forme de chloroacétate;
   b) l'hydrolyse du produit ainsi obtenu à l'aide d'une lipase appropriée, à une température d'environ 25-40° et un pH compris entre 5 et 8, et le traitement chromatographique du produit de la réaction, pour obtenir le chloroacétate de (+)-(1'S,1S,E)-1,2,2-triméthyl-4-(2',2',3'-triméthyl-3'-cyclopentén-1'-yl)-3-butényle et le (+)-(1'S,2R,E)-3,3-diméthyl-5-(2',2',3'-triméthyl-3'-cyclopentén-1'-yl)-4-pentén-2-ol; et

c) la saponification du chloroacétate de (+)-(1'S,1S,E)-1,2,2-triméthyl-4-(2',2',3'-triméthyl-3'-cyclopentén-1'-yl)-3-buténytle, à l'aide d'un hydroxyde de métal alcalin, pour obtenir le (+)-(1'S,2S,E)-3,3-diméthyl-5-(2',2',3'-triméthyl-3'-cyclopentén-1'-yl)-4-pentén-2-ol.

**9.** Procédé pour la préparation d'un composé selon la revendication 1 ayant une configuration (1'R,E), caractérisé en ce qu'il comprend les étapes suivantes:

a) l'estérification du (-)-(1'R,E)-3,3-diméthyl-5-(2',2',3'-triméthyl-3'-cyclopentén-1'-yl)-4-pentén-2-ol sous forme de chloroacétate;
b) l'hydrolyse du produit ainsi obtenu à l'aide d'une lipase appropriée, à une température d'environ 25-40° et un pH compris entre 5 et 8, et le traitement chromatographique du produit de la réaction, pour obtenir le chloroacétate de (-)-(1'R,1S,E)-1,2,2-triméthyl-4-(2',2',3'-triméthyl-3'-cyclopentén-1'-yl)-3-butényle et le (-)-(1'R,2R,E)-3,3-diméthyl-5-(2',2',3'-triméthyl-3'-cyclopentén-1'-yl)-4-pentén-2-ol; et
c) la saponification du chloroacétate de (-)-(1'R,1S,E)-1,2,2-triméthyl-4-(2',2',3'-triméthyl-3'-cyclopentén-1'-yl)-3-buténytle, à l'aide d'un hydroxyde de métal alcalin, pour obtenir le (-)-(1'R,2S,E)-3,3-diméthyl-5-(2',2',3'-triméthyl-3'-cyclopentén-1'-yl)-4-pentén-2-ol.

**10.** Procédé pour la préparation d'un composé selon la revendication 1, caractérisé en ce qu'on traite avec un excès de sodium métallique, dans l'éther, un céto-éther de formule

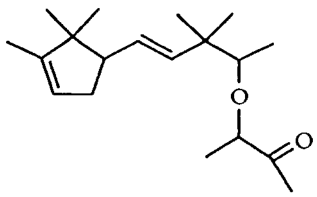

(I)

sous forme d'un isomère optiquement actif approprié, pour obtenir l'isomère correspondant du 3,3-diméthyl-5-(2,2,3-triméthyl-3-cyclopentén-1-yl)-4-pentén-2-ol.

**11.** Composé de formule (I) telle que définie à la revendication 10, sous forme d'un mélange d'isomères optiquement actifs ou de l'un quelconque de ces isomères.

## Claims

**1.** A compound selected from the group consisting of

a. (+)-(1'S,2R,E)-3,3-dimethyl-5-(2',2',3'-trimethyl-3'-cyclopenten-1'-yl)-4-penten-2-ol
b. (+)-(1'S,2S,E)-3,3-dimethyl-5-(2',2',3'-trimethyl-3'-cyclopenten-1'-yl)-4-penten-2-ol
c. (-)-(1'R,2R,E)-3,3-dimethyl-5-(2',2',3'-trimethyl-3'-cyclopenten-1'-yl)-4-penten-2-ol
d. (-)-(1'R,2S,E)-3,3-dimethyl-5-(2',2',3'-trimethyl-3'-cyclopenten-1'-yl)-4-penten-2-ol.

**2.** A compound selected from the group consisting of

a. (+)-(2S,E)-3,3-dimethyl-5-(2',2',3'-trimethyl-3'-cyclopenten-1'-yl)-4-penten-2-ol
b. (-)-(2R,E)-3,3-dimethyl-5-(2',2',3'-trimethyl-3'-cyclopenten-1'-yl)-4-penten-2-ol.

**3.** Use as a perfuming ingredient of a compound according to claim 1 or 2.

**4.** A perfuming composition or a perfumed article containing as an active ingredient a compound according to claim 1 or 2.

**5.** As a perfumed article of claim 4, a perfume or a cologne, a soap, a bath or shower gel, a cosmetic preparation, a shampoo, a body or ambient air deodorant, a detergent or a fabric softener, or a household product.

**6.** A method to confer, enhance, improve or modify the sandalwood-milky type odor character of a perfuming composition or a perfumed article, which method comprises adding to said composition or article a fragrance effective

amount of (-)-(1'R,E)-3,3-dimethyl-5-(2',2',3'-trimethyl-3'-cyclopenten-1'-yl)-4-penten-2-ol.

**7.** A method to confer, enhance, improve or modify the sandalwood-woody-cedar type odor character of a perfuming composition or a perfumed article, which method comprises adding to said composition or article a fragrance effective amount of (+)-(1'S,E)-3,3-dimethyl-5-(2,2',3'-trimethyl-3'-cyclopenten-1'-yl)-4-penten-2-ol.

**8.** A process for the preparation of a compound according to claim 1 having a (1'S,E) configuration characterized in that it comprises the following steps:

a) esterifying (+)-(1'S,E)-3,3-dimethyl-5-(2',2',3'-trimethyl-3'-cyclopenten-1'-yl)-4-penten-2-ol to form a chloroacetate derivative thereof;
b) hydrolysing the product thus obtained by means of an appropriate lipase, at a temperature of about 25-40° and a pH comprised between 5 and 8, and subjecting the reaction product to chromatographic separation to obtain (+)-(1'S,1S,E)-1,2,2-trimethyl-4-(2',2',3'-trimethyl-3'-cyclopenten-1'-yl)-3-butenyl chloroacetate and (+)-(1'S,2R,E)-3,3-dimethyl-5-(2',2',3'-trimethyl-3'-cyclopenten-1'-yl)-4-penten-2-ol ; and
c) saponifying said (+)-(1'S,1S,E)-1,2,2-trimethyl-4-(2',2',3'-trimethyl-3'-cyclopenten-1'-yl)-3-butenyl chloroacetate by means of an alkali metal hydroxide, to obtain (+)-(1'S,2S,E)-3,3-dimethyl-5-(2',2',3'-trimethyl-3'-cyclopenten-1'-yl)-4-penten-2-ol.

**9.** A process for the preparation of the compound according to claim 1 having a (1'R,E) configuration, characterized in that it comprises the following steps:

a) esterifying (-)-(1'R,E)-3,3-dimethyl-5-(2',2',3'-trimethyl-3'-cyclopenten-1'-yl)-4-penten-2-ol to form a chloroacetate derivative thereof;
b) hydrolysing the product thus obtained by means of an appropriate lipase, at a temperature of about 25-40° and a pH comprised between 5 and 8, and subjecting the reaction product to chromatographic separation to obtain (-)-(1'R,1S,E)-1,2,2-trimethyl-4-(2',2',3'-trimethyl-3'-cyclopenten-1'-yl)-3-butenyl chloroacetate and (-)-(1'R,2R,E)-3,3-dimethyl-5-(2',2',3'-trimethyl-3'-cyclopenten-1'-yl)-4-penten-2-ol ; and
c) saponifying said (-)-(1'R,1S,E)-1,2,2-trimethyl-4-(2',2',3'-trimethyl-3'-cyclopenten-1'-yl)-3-butenyl chloroacetate by means of an alkali metal hydroxide, to obtain (-)-(1'R,2S,E)-3,3-dimethyl-5-(2',2',3'-trimethyl-3'-cyclopenten-1'-yl)-4-penten-2-ol.

**10.** A process for the preparation of a compound according to claim 1, characterized in that there is treated with an excess of metallic sodium, in diethyl ether, a keto-ether of formula

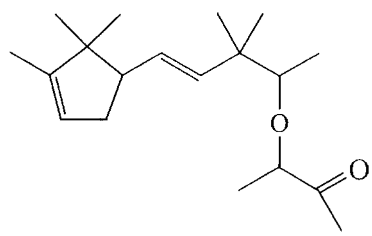

(I)

in the form of an appropriate optically active isomer thereof, to obtain the corresponding optically active isomer of 3,3-dimethyl-5-(2,2,3-trimethyl-3-cyclopenten-1-yl)-4-penten-2-ol.

**11.** A compound of formula (I) as defined in claim 10, in the form of a mixture of optically active isomers or of any one of these isomers.

**Patentansprüche**

**1.** Verbindung, ausgewählt aus der Gruppe bestehend aus

a. (+)-(1'S,2R,E)-3,3-Dimethyl-5-(2',2',3'-trimethyl-3'-cyclopenten-1'-yl)-4-penten-2-ol
b. (+)-(1'S,2S,E)-3,3-Dimethyl-5-(2',2',3'-trimethyl-3'-cyclopenten-1'-yl)-4-penten-2-ol
c. (-)-(1'R,2R,E)-3,3-Dimethyl-5-(2',2',3'-trimethyl-3'-cyclopenten-1'-yl)-4-penten-2-ol

d. (-)-(1'R,2S,E)-3,3-Dimethyl-5-(2',2',3'-trimethyl-3'-cyclopenten-1'-yl)-4-penten-2-ol.

**2.** Verbindung, ausgewählt aus der Gruppe bestehend aus

a. (+)-(2S,E)-3,3-Dimethyl-5-(2',2',3'-trimethyl-3'-cyclopenten-1'-yl)-4-penten-2-ol
b. (-)-(2R,E)-3,3-Dimethyl-5-(2',2',3'-trimethyl-3'-cyclopenten-1'-yl)-4-penten-2-ol.

**3.** Verwendung einer Verbindung gemäß Anspruch 1 oder 2 als Riechstoff.

**4.** Riechstoffzusammensetzung oder parfümierter Artikel, welche(r) eine Verbindung gemäß Anspruch 1 oder 2 als einen wirksamen Riechstoff enthält.

**5.** Als parfümierter Artikel gemäß Anspruch 4, ein Parfüm oder Eau de Toilette, eine Seife, ein Dusch- oder Badegel, ein kosmetisches Präparat, ein Shampoo, ein Körper- oder Raumluftdeodorant, ein Detergens oder Textilweichspüler oder ein Universalreiniger.

**6.** Verfahren zum Verleihen, Modifizieren, Verbessern oder Verstärken des sandelholz-/sandelholzmilchartigen Geruchscharakters einer Riechstoffzusammensetzung oder eines parfümierten Artikels, dadurch gekennzeichnet, daß der Zusammensetzung bzw. dem Artikel (-)-(1'R,E)-3,3-Dimethyl-5-(2','2',3'-trimethyl-3'-cyclopenten-1'-yl)-4-penten-2-ol zugegeben wird.

**7.** Verfahren zum Verleihen, Modifizieren, Verbessern oder Verstärken des sandelholz-/holz-/cedernartigen Geruchscharakters einer Riechstoffzusammensetzung oder eines parfümierten Artikels, dadurch gekennzeichnet, daß der Zusammensetzung bzw. dem Artikel (+)-(1'S,E)-3,3-Dimethyl-5-(2',2',3'-trimethyl-3'-cyclopenten-1'-yl)-4-penten-2-ol zugegeben wird.

**8.** Verfahren zur Herstellung einer Verbindung gemäß Anspruch 1 mit einer (1'S,E)-Konfiguration, dadurch gekennzeichnet, daß es die folgenden Schritte aufweist:

a) Verestern von (+)-(1'S,E)-3,3-Dimethyl-5-(2','2',3'-trimethyl-3'-cyclopenten-1'-yl)-4-penten-2-ol in Form von Chloracetat;
b) Hydrolyse des auf diese Weise erhaltenen Produkts mit Hilfe einer geeigneten Lipase bei einer Temperatur von ca. 25-40° und einem zwischen 5 und 8 liegenden pH-Wert, sowie die chromatographische Behandlung des Reaktionsprodukts, um das Chloracetat von (+)-(1'S,1S,E)-1,2,2-Trimethyl-4-(2',2',3'-trimethyl-3'-cyclopenten-1'-yl)-3-butenyl und (+)-(1'S,2R,E)-3,3-Dimethyl-5-(2',2',3'-trimethyl-3'-cyclopenten-1'-yl)-4-penten-2-ol zu erhalten; und
c) Verseifen des Chloracetats von (+)-(1'S,1S,E)-1,2,2-Trimethyl-4-(2',2',3'-trimethyl-3'-cyclopenten-1'-yl)-3-butenyl mit Hilfe eines Hydroxids eines Alkalimetalls, um (+)-(1'S,2S,E)-3,3-Dimethyl-5-(2',2',3'-trimethyl-3'-cyclopenten-1'-yl)-4-penten-2-ol zu erhalten.

**9.** Verfahren zur Herstellung einer Verbindung gemäß Anspruch 1 mit einer (1'R,E)-Konfiguration, dadurch gekennzeichnet, daß es die folgenden Schritte aufweist:

a) Verestern von (-)-(1'R,E)-3,3-Dimethyl-5-(2',2',3'-trimethyl-3'-cyclopenten-1'-yl)-4-penten-2-ol in Form von Chloracetat;
b) Hydrolyse des auf diese Weise erhaltenen Produkts mit Hilfe einer geeigneten Lipase bei einer Temperatur von ca. 25-40° und einem zwischen 5 und 8 liegenden pH-Wert, sowie die chromatographische Behandlung des Reaktionsprodukts, um das Chloracetat von (-)-(1'R,1S,E)-1,2,2-Trimethyl-4-(2',2',3'-trimethyl-3'-cyclopenten-1'-yl)-3-butenyl und (-)-(1'R,2R,E)-3,3-Dimethyl-5-(2',2','3,-trimethyl-3'-cyclopenten-1'-yl)-4-penten-2-ol zu erhalten; und
c) Verseifen des Chloracetats von (-)-(1'R,1S,E)-1,2,2-Trimethyl-4-(2',2',3'-trimethyl-3'-cyclopenten-1'-yl)-3-butenyl mit Hilfe eines Hydroxids eines Alkalimetalls, um (-)-(1'R,2S,E)-3,3-Dimethyl-5-(2',2',3'-trimethyl-3'-cyclopenten-1'-yl)-4-penten-2-ol zu erhalten.

**10.** Verfahren zur Herstellung einer Verbindung gemäß Anspruch 1, dadurch gekennzeichnet, daß man einen Ketoether mit der Formel

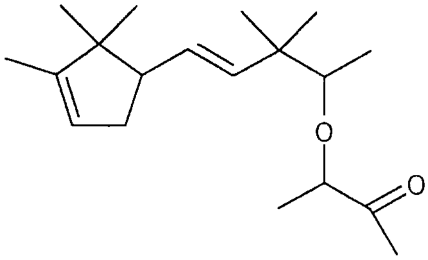

(I)

in Form eines geeigneten, optisch aktiven Isomers mit einem Überschuß an metallischem Natrium in Ether behandelt, um das entsprechende Isomer von 3,3-Dimethyl-5-(2,2,3-trimethyl-3-cyclopenten-1-yl)-4-penten-2-ol zu erhalten.

**11.** Verbindung mit der Formel (I) gemäß der Definition von Anspruch 10 in Form einer Mischung von optisch aktiven Isomeren oder eines dieser Isomere.